# EUROPÄISCHE PATENTSCHRIFT

(11) **EP 0 731 089 B1**
(45) Veröffentlichungstag und Bekanntmachung des Hinweises auf die Patenterteilung: **20.10.1999**
(21) Anmeldenummer: 96103235.6
(22) Anmeldetag: 02.03.1996
(51) Int. Cl.: C07C 317/36, C07C 317/40

(54) **Anilinderivate**
Aniline derivatives
Dérivés d'aniline

(30) Priorität: 09.03.1995 DE 19508311
(43) Veröffentlichungstag der Anmeldung: 11.09.1996
(73) Patentinhaber: BASF AKTIENGESELLSCHAFT, 67056 Ludwigshafen (DE)
(72) Erfinder: Patsch, Manfred, Dr., 67157 Wachenheim (DE)

(56) Entgegenhaltungen:
- EP-A- 0 071 168
- EP-A- 0 180 732
- EP-A- 0 213 485
- DE-A- 2 634 857
- DE-B- 1 246 906

## Beschreibung

Die vorliegende Erfindung betrifft neue Aniline der Formel I 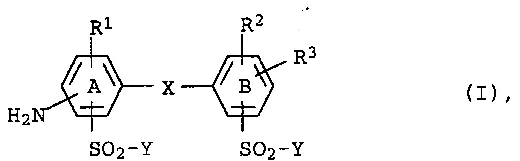 in der
Ringe A und B jeweils benzoanelliert sein können und
- X: einen Rest der Formel CO, SO₂, CONZ oder SO₂NZ, worin Z jeweils für Wasserstoff oder C₁-C₄-Alkyl steht,
- R¹ und R²: unabhängig voneinander jeweils Wasserstoff, C₁-C₄-Alkyl, C₁-C₄-Alkoxy, Halogen, Hydroxysulfonyl, Carboxyl, C₁-C₄-Alkoxycarbonyl oder C₁-C₄-Alkylsulfonyl,
- R³: Wasserstoff, Amino, C₁-C₄-Alkanoylamino oder Nitro und
- Y: Vinyl oder einen Rest der Formel C₂H₄-Q, worin Q für eine unter alkalischen Reaktionsbedingungen abspaltbare Gruppe steht,
bedeuten.

DE-B-1 246 906 und DE-A-2 634 857 lehren Aniline, die sich aus zwei verbrückten Phenylringen, von denen einer einen faserreaktiven Rest trägt, aufbauen.

Aufgabe der vorliegenden Erfindung war es, neue Anilinderivate bereitzustellen, die auf einfache Weise herzustellen sind und als Zwischenprodukte für Farbstoffe dienen können.

Demgemäß wurden die eingangs näher bezeichneten Aniline der Formel I gefunden.

Alle in der obengenannten Formel I auftretenden Alkylreste können sowohl geradkettig als auch verzweigt sein.

Reste Z, R¹ und R² sind z.B. Methyl, Ethyl, Propyl, Isopropyl, Butyl, Isobutyl, sec-Butyl oder tert-Butyl.

Reste R¹ und R² sind weiterhin z.B. Methoxy, Ethoxy, Propoxy, Isopropoxy, Butoxy, Isobutoxy, sec-Butoxy, Fluor, Chlor, Brom, Methoxycarbonyl, Ethoxycarbonyl, Propoxycarbonyl, Isopropoxycarbonyl, Butoxycarbonyl, Methylsulfonyl, Ethylsulfonyl, Propylsulfonyl, Isopropylsulfonyl oder Butylsulfonyl.

Reste R³ sind z.B. Formylamino, Acetylamino, Propionylamino, Butyrylamino oder Isobutyrylamino.

Der Rest Q steht für eine unter alkalischen Reaktionsbedingungen abspaltbare Gruppe. Solche Gruppen sind z.B. Chlor, Brom, C₁-C₄-Alkylsulfonyl, Phenylsulfonyl, OSO₃H, SSO₃H, OP(O)(OH)₂, C₁-C₄-Alkylsulfonyloxy, Phenylsulfonyloxy, C₁-C₄-Alkanoyloxy, C₁-C₄-Dialkylamino oder ein Rest der Formel 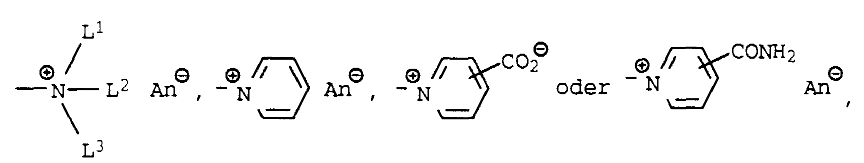 wobei L¹, L² und L³ unabhängig voneinander jeweils die Bedeutung von C₁-C₄-Alkyl oder Benzyl und An^{⊖} jeweils die Bedeutung eines Äquivalents eines Anions besitzen. Als Anionen können dabei z.B. Fluorid, Chlorid, Bromid, Iodid, Mono-, Di- oder Trichloracetat, Methansulfonat, Benzolsulfonat oder 2- oder 4-Methylbenzolsulfonat in Betracht kommen.

Wenn in den Anilinen der Formel I Hydroxysulfonyl- und/oder Carboxylgruppen auftreten, so werden neben den freien Säuren selbstverständlich auch deren Salze von den Patentansprüchen umfaßt.

Geeignete Kationen leiten sich dabei von Metall- oder Ammoniumionen ab. Metallionen sind insbesondere die Lithium-, Natrium- oder Kaliumionen. Unter Ammoniumionen im erfindungsgemäßen Sinne sind unsubstituierte oder substituierte Ammoniumkationen zu verstehen. Substituierte Ammoniumkationen sind z.B. Monoalkyl-, Dialkyl-, Trialkyl-, Tetraalkyl- oder Benzyltrialkylammoniumkationen oder solche Kationen, die sich von stickstoffhaltigen fünf- oder sechsgliedrigen gesättigten Heterocyclen ableiten, wie Pyrrolidinium-, Piperidinium-, Morpholinium-, Piperazinium- oder N-Alkylpiperaziniumkationen oder deren N-monoalkyl- oder N,N-dialkylsubstituierte Produkte. Unter Alkyl ist dabei im allgemeinen geradkettiges oder verzweigtes C₁-C₂₀-Alkyl zu verstehen, das durch Hydroxylgruppen substituiert und/oder durch 1 bis 4 Sauerstoffatome in Etherfunktion unterbrochen sein kann.

Bevorzugt sind Aniline der Formel I, in der die Ringe A und B jeweils nicht benzoanelliert sind.

Weiterhin bevorzugt sind Aniline der Formel I, in der X einen Rest der Formel CO, SO₂, CONH oder SO₂NH bedeutet.

Weiterhin bevorzugt sind Aniline der Formel I, in der R¹ und R² jeweils Wasserstoff bedeuten.

Weiterhin bevorzugt sind Aniline der Formel I, in der R³ Wasserstoff, Amino oder Acetylamino bedeutet.

Weiterhin bevorzugt sind Aniline der Formel I, in der im Ring A die Aminogruppe und der Rest S02-Y in ortho-Position zueinander stehen.

Besonders bevorzugt sind Aniline der Formel Ia 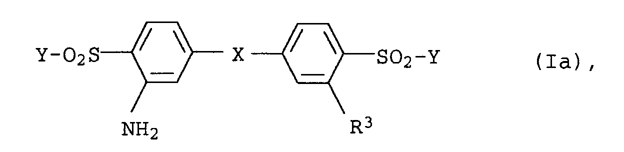 in der
- X: einen Rest der Formel CO, SO₂ oder SO₂NH und
- R³: Wasserstoff oder Amino bedeuten und
- Y: die obengenannte Bedeutung besitzt.

Die erfindungsgemäßen Aniline der Formel I können nach an sich bekannten Methoden hergestellt werden.

Beispielsweise kann man eine Halogenverbindung der Formel II 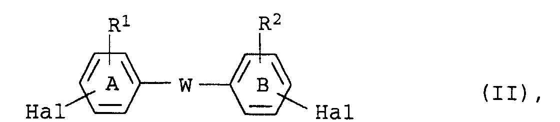 in der Hal jeweils Halogen, vorzugsweise Chlor, und W einen Rest der Formel CO oder SO₂ bedeuten und R¹ und R² sowie die Ringe A und B jeweils die obengenannte Bedeutung besitzen, auf an sich üblichem Wege nitrieren und die resultierende Nitroverbindung der Formel III 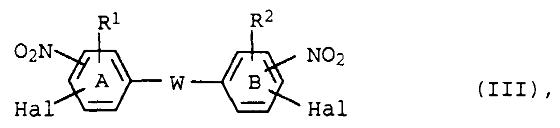 in der Hal, R¹, R², W und die Ringe A und B jeweils die obengenannte Bedeutung besitzen, mit 2-Thioethanol umsetzen, wobei man die Verbindung der Formel IV 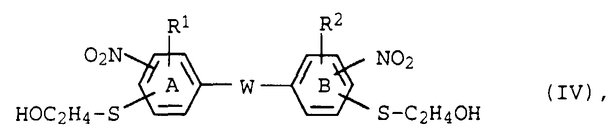 erhält, in der R¹, R², W und die Ringe A und B jeweils die obengenannte Bedeutung besitzen.

Aus der Verbindung IV können dann durch Reduktion der Nitrogruppe zur Aminogruppe, Oxidation des Schwefelatoms zur Sulfonylgruppe und Überführung des Rests C₂H₄OH in die oben näher bezeichnete Gruppe Y, wobei diese Schritte in beliebiger Reihenfolge vorgenommen werden können, diejenigen erfindungsgemäßen Aniline der Formel I erhalten werden, in der X einen Rest der Formel CO oder SO₂ bedeutet.

Diejenigen Aniline der Formel I, in der X einen Rest der Formel CONZ oder SO₂NZ bedeutet, können z.B. durch Umsetzung der Säurehalogenide der Formel V 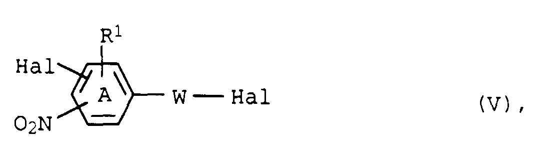 in der Hal, R¹, W und der Ring A jeweils die obengenannte Bedeutung besitzen, mit Aminen der Formel VI 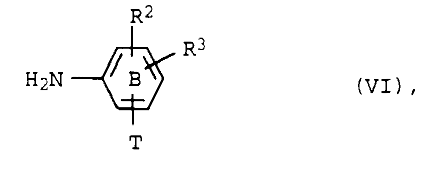 in der R², R³ und der Ring B jeweils die obengenannte Bedeutung besitzen und T 2-Hydroxyethylthio oder den obengenannten Rest S02-Y bedeutet, und anschließender Reduktion der Nitrogruppe zur Aminogruppe, Oxidation des Schwefelatoms zur Sulfonylgruppe und Überführung des Rests C₂H₄OH in die oben näher bezeichnete Gruppe Y erhalten werden, wobei die letzten drei Schritte wiederum in beliebiger Reihenfolge vorgenommen werden können.

Bei den neuen Anilinen der Formel I handelt es sich um wertvolle Zwischenprodukte für Farbstoffe, insbesondere Reaktivfarbstoffe.

Die folgenden Beispiele sollen die Erfindung näher erläutern.

### Beispiel 1

Zu 997 g 4,4'-Dichlor-3,3'-dinitrobenzophenon in 3000 ml Butanol gab man 546 g 2-Mercaptoethanol und 336 g Natriumhydrogencarbonat und erhitzte 12 Stunden auf 110°C. Danach ließ man abkühlen und saugte den entstandenen Niederschlag ab. Nach Waschen mit Isopropanol und anschließend mit heißem Wasser erhielt man nach Trocknung im Vakuumtrockenschrank 798 g 4,4'-Bis(2-hydroxyethylmercapto)-3,3'-dinitrobenzophenon. Fp.: 193 bis 197°C.

### Beispiel 2

a) 212 g der Verbindung aus Beispiel 1 wurden in einer Mischung aus 2500 ml Isopropanol und 1000 ml Wasser gelöst. Man gab 50 ml Eisessig und 5 g Palladium auf Kohle zu und begaste bei 40 bis 50°C mit Wasserstoff. Nach beendeter Wasserstoffaufnahme wurde vom Katalysator abfiltriert und die Mutterlauge am Rotationsverdampfer eingeengt. Man isolierte 180 g eines braunen Öls.
b) 36,4 g des unter a) erhaltenen Öls wurden in 50 ml Aceton und 50 ml Wasser mit 25 g Acetanhydrid bei 45 bis 50°C umgesetzt. Nach Zugabe von 1 g Wolframsäure wurden innerhalb von 3 Stunden 48 g 30 gew.-%iges wäßriges Wasserstoffperoxid zugetropft. Nach Einengen und Umkristallisation aus Butanol isolierte man 25 g 4,4'-Bis(2-hydroxyethylsulfonyl)-3,3'-bis-acetylaminobenzophenon. Fp.: 189 bis 192°C.

### Beispiel 3

180 g der Verbindung aus Beispiel 2b) wurden in 200 ml 20 gew.-%iger Salzsäure 2 Stunden auf 100°C erhitzt. Man goß auf 1000 ml Wasser und gab verdünnte Natronlauge zu, bis ein pH-Wert von 6 erreicht war. Dabei schied sich ein braunes Öl ab. Nach Trocknung isolierte man 120 g 4,4'-Bis(2-hydroxyethylsulfonyl)-3,3'-diaminobenzophenon.

### Beispiel 4

Man trug 75 g der Verbindung aus Beispiel 3 bei 15 bis 20°C in 500 g 5 gew.-%iges Oleum ein und rührte das Gemisch weitere 24 Stunden. Dann wurde mit Wasser auf 1200 g verdünnt. Es schied sich ein Niederschlag ab, den man absaugte. Man isolierte 91 g der Verbindung der Formel 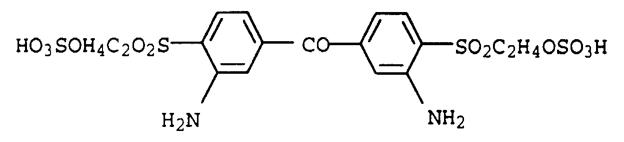 Fp.: 203 bis 105°C

### Beispiel 5

Man arbeitete wie in den Beispielen 1 bis 4, verwendete jedoch als Ausgangsprodukt eine äquivalente Menge an 4,4'-Dichlor-3,3'-dinitrodiphenylsulfon, und erhielt über die Zwischenstufen der Formel

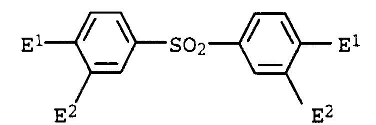

| E¹ | E² | Fp. |
|---|---|---|
| SC₂H₄OH | NO₂ | 296-300°C |
| SC₂H₄OH | NH₂ | Öl |
| SO₂C₂H₄OH | NHCOCH₃ | 206-210°C |
| SO₂C₂H₄OH | NH₂ | 234-237°C |

das Produkt mit E¹ = SO₂C₂H₄OSO₃H und E² = NH₂ (Fp > 230°C Zers.).

### Beispiel 6

285 g 3-Nitro-4-chlorbenzolsulfonsäurechlorid und 242 g 4-(2-Hydroxyethylsulfonyl)anilin wurden in 750 ml Wasser bei einem pH-Wert von 6,5 6 Stunden bei 40°C gerührt. Der Niederschlag wurde abgesaugt. Nach Trocknung isolierte man 480 g der Verbindung der Formel 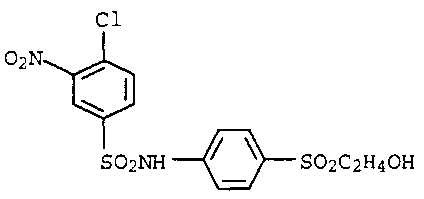 Fp.: 147 bis 152°C.

### Beispiel 7

430 g der Verbindung aus Beispiel 6 wurden in 750 ml Isobutanol mit 77 g 2-Mercaptoethanol und 84 g Natriumhydrogencarbonat 6 Stunden unter Rückfluß erhitzt. Man isolierte 370 g der Verbindung der Formel 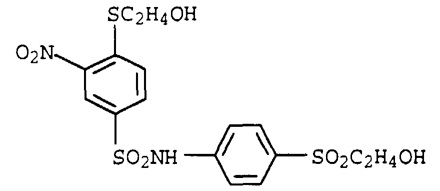 NMR (D₆-DMSO): 3,20(M,2), 3,35(M,2), 3,69 (M,4) ∼ 5,0(br.S,2), 7,28(D,2), 7,73(D,2), 7,86(D,1), 8,02(D,1), 8,58(D,1).

### Beispiel 8

a) 370 g der Verbindung aus Beispiel 7 wurden in einer Mischung aus 1000 ml Wasser, 1000 ml Isopropanol und 500 ml Eisessig bei 60 bis 65°C mit 113 g Eisenpulver versetzt. Die Temperatur stieg dabei bis 80°C an. Nach Klärfiltration wurde das Lösungsmittel unter vermindertem Druck abdestilliert. Es verblieben 205 g eines braunen Öls.
b) 172 g der unter a) beschriebenen Verbindung in 200 ml Wasser wurden bei 40 bis 50°C mit 100 ml Acetanhydrid versetzt. Nach 3 Stunden Rühren bei 60 bis 65°C wurde auf 200 ml Wasser gegossen, 0,5 g Wolframsäure zugegeben und bei 60 bis 65°C 91 g 30 gew.-%iges Wasserstoffperoxid zugetropft. Der entstandene Niederschlag wurde abgesaugt. Man isolierte 116 g der Verbindung der Formel 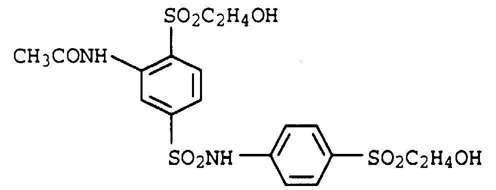 Fp.: 134 bis 136°C.
c) Entacetylierung und Bildung des Bisschwefelsäurehalbesters erfolgte in konz. Schwefelsäure bei 35 bis 40°C. Nach Verdünnen auf Eis erhielt man eine Lösung der Verbindung der Formel 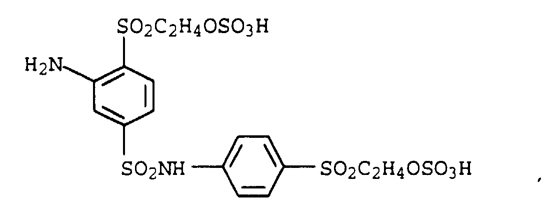 die direkt zur Farbstoffsynthese verwendet werden kann.

## Patentansprüche

1. Aniline der Formel I 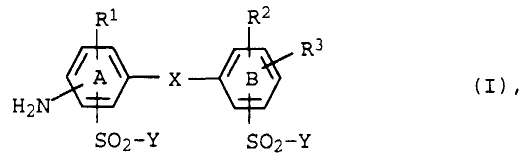 in der
Ringe A und B jeweils benzoanelliert sein können und
X einen Rest der Formel CO, SO₂, CONZ oder SO₂NZ, worin Z jeweils für Wasserstoff oder C₁-C₄-Alkyl steht,
R¹ und R² unabhängig voneinander jeweils Wasserstoff, C₁-C₄-Alkyl, C₁-C₄-Alkoxy, Halogen, Hydroxysulfonyl, Carboxyl, C₁-C₄-Alkoxycarbonyl oder C₁-C₄-Alkylsulfonyl,
R³ Wasserstoff, Amino, C₁-C₄-Alkanoylamino oder Nitro und
Y Vinyl oder einen Rest der Formel C₂H₄-Q, worin Q für eine unter alkalischen Reaktionsbedingungen abspaltbare Gruppe steht,
bedeuten.

2. Aniline nach Anspruch 1, dadurch gekennzeichnet, daß die Ringe A und B jeweils nicht benzoanelliert sind.

3. Aniline nach Anspruch 1, dadurch gekennzeichnet, daß X einen Rest der Formel CO, SO₂, CONH oder SO₂NH bedeutet.

4. Aniline nach Anspruch 1, dadurch gekennzeichnet, daß R¹ und R² jeweils Wasserstoff bedeuten.

5. Aniline nach Anspruch 1, dadurch gekennzeichnet, daß R³ Wasserstoff, Amino oder Acetylamino bedeutet.

6. Aniline nach Anspruch 1, dadurch gekennzeichnet, daß im Ring A die Aminogruppe und der Rest SO₂-Y in ortho-Position zueinander stehen.

## Claims

1. Anilines of the formula I 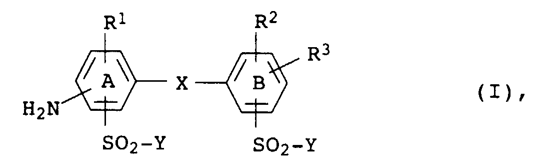 where
rings A and B may each be benzofused,
X is a radical of the formula CO, SO₂, CONZ or SO₂NZ, where Z is in each case hydrogen or C₁-C₄-alkyl,
R¹ and R² are each independently of the other hydrogen, C₁-C₄-alkyl, C₁-C₄-alkoxy, halogen, hydroxysulfonyl, carboxyl, C₁-C₄-alkoxycarbonyl or C₁-C₄-alkylsulfonyl,
R³ is hydrogen, amino, C₁-C₄-alkanoylamino or nitro, and
Y is vinyl or a radical of the formula C₂H₄-Q, where Q is a group detachable under alkaline reaction conditions.

2. Anilines as claimed in claim 1, wherein said rings A and B are each not benzofused.

3. Anilines as claimed in claim 1, wherein X is a radical of the formula CO, SO₂, CONH or SO₂NH.

4. Anilines as claimed in claim 1, wherein R¹ and R² are each hydrogen.

5. Anilines as claimed in claim 1, wherein R³ is hydrogen, amino or acetylamino.

6. Anilines as claimed in claim 1, wherein, in ring A, the amino group and the SO₂-Y radical are ortho to each other.

## Revendications

1. Aniline de formule I 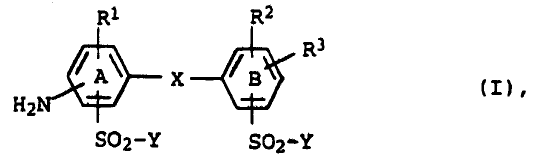 dans laquelle
les cycles A et B peuvent chacun être benzocondensés et
X représente un reste de formule CO, SO₂, CONZ ou SO₂NZ, où Z est à chaque fois mis pour un atome d'hydrogène ou un groupement alkyle en C₁-C₄,
R¹ et R² représentent chacun, indépendamment l'un de l'autre, un atome d'hydrogène, un groupement alkyle en C₁-C₄, alcoxy en C₁-C₄, un atome d'halogène, un groupement hydroxysulfonyle, carboxyle, alcoxycarbonyle en C₁-C₄ ou alkylsulfonyle en C₁-C₄,
R³ représente un atome d'hydrogène, un groupement amino, un groupement alcanoylamino en C₁-C₄ ou nitro et
Y représente un groupement vinyle ou un reste de formule C₂H₄-Q, où Q est mis pour un groupement séparable dans des conditions réactionnelles alcalines.

2. Aniline selon la revendication 1, caractérisée en ce que les cycles A et B ne sont pas benzocondensés.

3. Aniline selon la revendication 1, caractérisée en ce que X représente un reste de formule CO, SO₂, CONH ou SO₂NH.

4. Aniline selon la revendication 1, caractérisée en ce que R¹ et R² représentent chacun un atome d'hydrogène.

5. Aniline selon la revendication 1, caractérisée en ce que R³ représente un atome d'hydrogène, un groupement amino ou acétylamino.

6. Aniline selon la revendication 1, caractérisée en ce que, dans le cycle A, le groupement amino et le reste SO₂-Y sont en position ortho, l'un par rapport à l'autre.
